(19) Europäisches Patentamt
European Patent Office
Office européen des brevets

(11) Veröffentlichungsnummer: **0 114 609**
**B1**

(12) # EUROPÄISCHE PATENTSCHRIFT

(45) Veröffentlichungstag der Patentschrift: **23.03.88**

(51) Int. Cl.⁴: **C 07 D 249/08,** A 01 N 43/653

(21) Anmeldenummer: **84100178.7**

(22) Anmeldetag: **10.01.84**

(54) **(-)-Antipode des (E)-1-Cyclohexyl-4,4-dimethyl-3-hydroxy-2-(1,2,4-triazol-1-yl)-pent-1-ens.**

(30) Priorität: **22.01.83 DE 3302122**

(43) Veröffentlichungstag der Anmeldung: **01.08.84 Patentblatt 84/31**

(45) Bekanntmachung des Hinweises auf die Patenterteilung: **23.03.88 Patentblatt 88/12**

(84) Benannte Vertragsstaaten: **AT BE CH DE FR GB IT LI NL**

(56) Entgegenhaltungen:
**EP-A-0 015 387**
**EP-A-0 142 566**

(73) Patentinhaber: **BAYER AG**
**Konzernverwaltung RP Patentabteilung**
**D-5090 Leverkusen 1 Bayerwerk (DE)**

(72) Erfinder: **Kraatz, Udo, Dr.**
**Andreasstrasse 22a**
**D-5090 Leverkusen 1 (DE)**
Erfinder: **Reiser, Wolf, Dr.**
**Kiebitzweg 12a**
**D-5600 Wuppertal 1 (DE)**
Erfinder: **Büchel, Karl Heinz, Prof. Dr.**
**Dabringhausener Strasse 42**
**D-5093 Burscheid (DE)**
Erfinder: **Lürssen, Klaus, Dr.**
**August-Kierspel-Strasse 89**
**D-5060 Bergisch Glasbach 2 (DE)**

**EP 0 114 609 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft den neuen (−)-Antipoden*) des (E)-1-Cyclohexyl-4,4-dimethyl-3-hydroxy-2-(1,2,4-triazol-1-yl)-pent-1-ens, ein Verfahren zu dessen Herstellung und dessen Verwendung zur Regulierung des Pflanzenwachstums.

Es ist bereits bekannt, daß das Racemat des 1-Cyclohexyl-4,4-dimethyl-3-hydroxy-2-(1,2,4-triazol-1-yl)-pent-1-ens pflanzenwuchsregulierende Eigenschaften besitzt (vgl. DE—OS 29 06 061). Die Wirksamkeit dieses Produktes ist gut, jedoch ist der bei sehr niedrigen Aufwandmengen erzielte Effekt nicht immer befriedigend.

Es wurde nun der (−)-Antipode des (E)-1-Cyclohexyl-4,4-dimethyl-3-hydroxy-2-(1,2,4-triazol-1-yl)-pent-1-ens der Formel

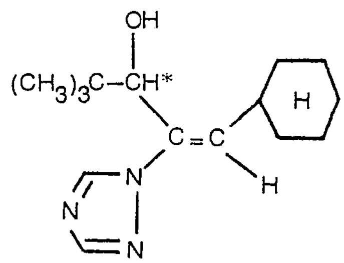

(I)

gefunden.

Weiterhin wurde gefunden, daß man den neuen (−)-Antipoden des (E)-1-Cyclohexyl-4,4,-dimethyl-3-hydroxy-2-(1,2,4-triazol-1-yl)-pent-1-ens der Formel (I) erhält, wenn man in einer ersten Stufe racemisches (E)-1-Cyclohexyl-4,4-dimethyl-3-hydroxy-2-(1,2,4-triazol-1-yl)-pent-1-en der Formel

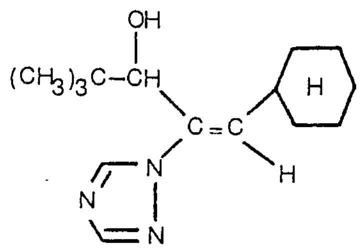

(Ia)

mit einem optisch aktiven Säurehalogenid der Formel in welcher

R für (+)-3-Brom-campher-8-yl, (+)-Campher-10-yl oder (−)-Menth-3-yl steht,

X für —CO—, —$SO_2$— oder —O—$CH_2$—CO— steht und

Hal für Chlor oder Brom steht,

in Gegenwart eines Verdünnungsmittels und gegebenenfalls in Gegenwart von Basen umsetzt, dann die so erhaltenen diastereomeren Ester der Formel

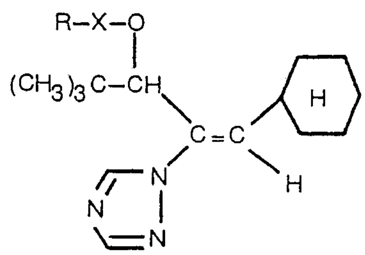

(III)
(Diastereomeren-Gemisch)

in welcher

R und X die oben angegebene Bedeutung haben,

aufgrund ihrer unterschiedlichen physikalischen Eigenschaften trennt

und danach in einer zweiten Stufe den (−)-Antipoden des (E)-1-Cyclohexyl-4,4-dimethyl-3-hydroxy-2-(1,2,4-triazol-1-yl)-pent-1-ens aus dem entsprechenden Ester mit Hilfe von Basen in Gegenwart eines Verdünnungsmittels in Freiheit setzt.

Schließlich wurde gefunden, daß sich der neue (−)-Antipode des (E)-1-Cyclohexyl-4,4-dimethyl-3-hydroxy-2-(1,2,4-triazol-1-yl)-pent-1-ens der Formel (I) durch eine hervorragende pflanzenwuchsregulierende Wirksamkeit auszeichnet.

---

*) Unter dem (−)-Antipoden ist hier jeweils dasjenige Enantiomere zu verstehen, das die Schwingungsebene von linear polarisiertem Licht der Natrium-D-Linie nach links dreht.

Überraschenderweise besitzt der neue (−)-Antipode des (E)-1-Cyclohexyl-4,4-dimethyl-3-hydroxy-2-(1,2,4-triazol-1-yl)-pent-1-ens der Formel (I) wesentlich bessere pflanzenwuchsregulierende Eigenschaften als das entsprechende Racemat, das aus dem Stand der Technik als hoch wirksamer Pflanzenwuchsregulator bekannt ist. Im übringen konnte nicht erwartet werden, daß der erfindungsgemäße Wirkstoff sich durch sehr gute pflanzenwuchsregulierende Wirksamkeit hervorhebt, während der (+)-Antipode des (E)-1-Cyclohexyl-4,4-dimethyl-3-hydroxy-2-(1,2,4-triazol-1-yl)-pent-1-ens als Pflanzenwuchsregulator weitgehend inaktiv ist.

Die erfindungsgemäße Verbindung ist durch die Formel (I) definiert. In dieser Formel ist das asymmetrische Kohlenstoffatom durch ein (*) gekennzeichnet.

Verwendet man das racemische (E)-1-Cyclohexyl-4,4-dimethyl-3-hydroxy-2-(1,2,4-triazol-1-yl)-pent-1-ens als Ausgangsstoff, (−)-Menth-3-yloxy-acetylchlorid als optisch aktives Säurehalogenid, Triethylamin und 4-Dimethylaminopyridin (DMPA) als Hilfsbasen zur Veresterung (1. Stufe) und ein Gemisch aus Natriumhydroxid, Wasser und Methanol zur Esterverseifung (2. Stufe), so kann der Reaktionsablauf des erfindungsgemäßen Verfahrens durch das folgende Formelschema wiedergegeben werden:

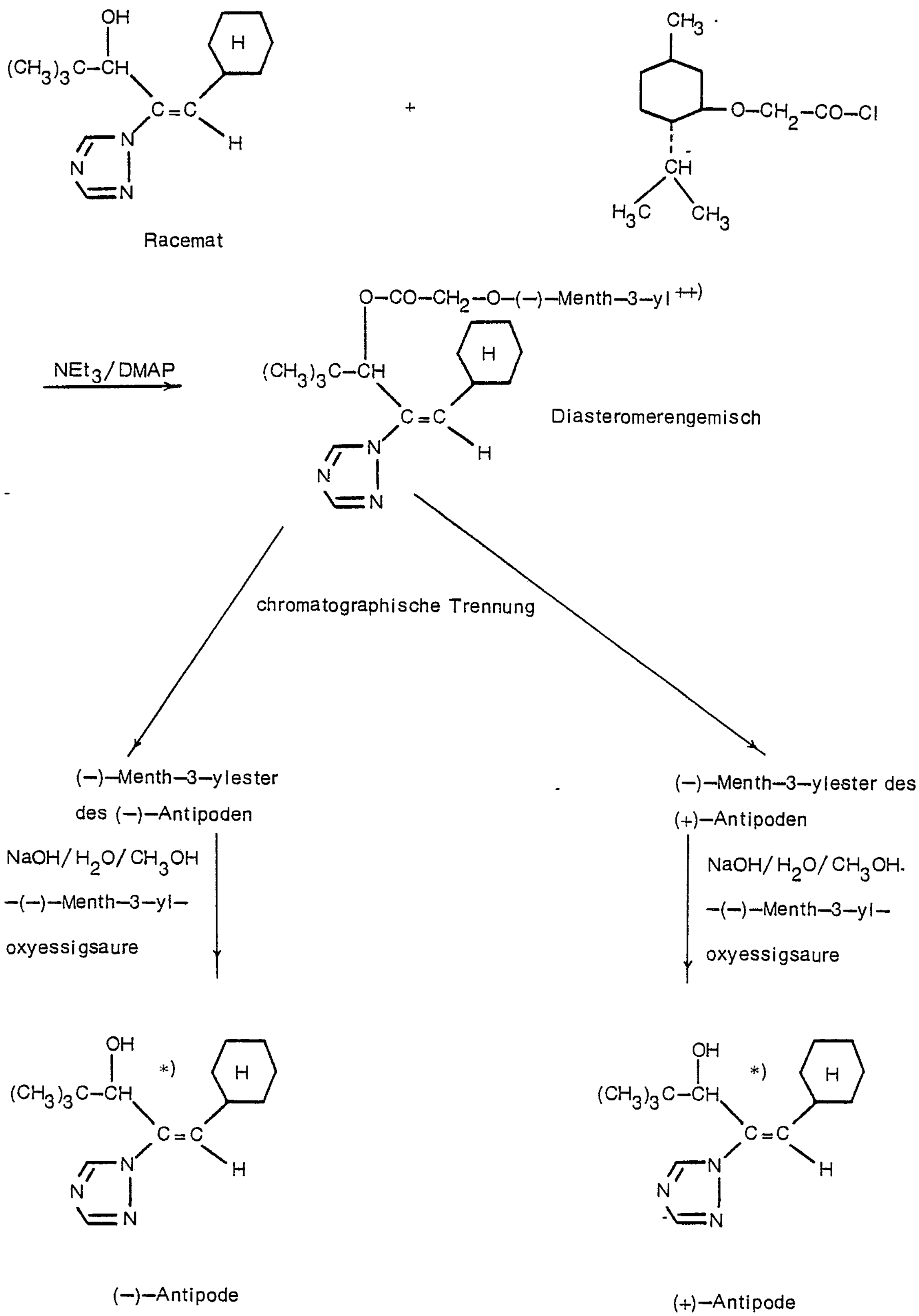
Racemat
+
O–CO–CH2–O–(–)–Menth–3–yl ++)
NEt3/DMAP
Diasteromerengemisch
chromatographische Trennung
(–)–Menth–3–ylester
des (–)–Antipoden
NaOH/H2O/CH3OH
–(–)–Menth–3–yl–
oxyessigsaure
(–)–Menth–3–ylester des
(+)–Antipoden
NaOH/H2O/CH3OH.
–(–)–Menth–3–yl–
oxyessigsaure
*)
*)
(–)–Antipode
(+)–Antipode

---

**) (–)–Menth–3–yl = 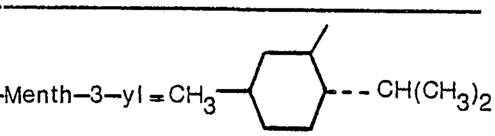

Das bei der Durchführung des erfindungsgemäßen Verfahrens als Ausgangsstoff benötigte Racemat des (E)-1-Cyclohexyl-4,4-dimethyl-3-hydroxy-2-(1,2,4-triazol-1-yl)-pent-1-ens der Formel (Ia) ist bekannt (vgl. DE—OS 29 06 061).

Die bei dem erfindungsgemäßen Verfahren weiterhin als Ausgangssubstanzen benötigten optisch aktiven Säurehalogenide sind durch die Formel (II) allgemein definiert.

Als Beispiele für Verbindungen der Formel (II) seien genannt:

(+)-3-Brom-campher-8-sulfonsäurechlorid
(+)-Campher-10-sulfonsäurechlorid
(+)-3-Brom-campher-8-sulfonsäurebromid
(+)-Campher-10-sulfonsäurebromid
(−)-Menth-3-yl-oxyacetylchlorid
(−)-Menth-3-yl-oxyacetylbromid

Die optisch aktiven Säurehalogenide sind bekannt oder lassen sich nach bekannten Methoden in einfacher Weise herstellen.

Als Verdünnungsmittel kommen für die Durchführung der 1. Stufe (Veresterung) des erfindungsgemäßen Verfahrens alle inerten organischen Lösungsmittel in Frage. Hierzu gehören vorzugsweise Kohlenwasserstoffe, wie Benzin, Benzol, Toluol oder Xylol, halogenierte Kohlenwasserstoffe, wie Dichlormethan, Chloroform, Tetrachlorkohlenstoff, Chlorbenzol oder Dichlorbenzol, Ether wie Diethylether, Tetrahydrofuran oder Dioxan, Nitrile, wie Acetonitril oder Propionitril oder Ester, wie Essigsäureethylester.

Die erste Stufe des erfindungsgemäßen Verfahrens wird vorzugsweise in Gegenwart von Basen durchgeführt. Hierbei können alle üblichen organischen oder anorganischen Basen eingesetzt werden. Vorzugsweise verwendbar sind Alkalihydroxide oder Alkalicarbonate, wie beispielsweise Natriumhydroxid, Natriumcarbonat oder Natriumhydrogencarbonat, ferner niedere tertiäre Alkylamine, Cycloalkylamine, Arylalkylamine oder Arylamine, wie beispielsweise Triethylamin, N,N-Dimethylbenzylamin, Pyridin, 1,4-Diazabicyclo-[2,2,2]-octan oder 1,5-Diazabicyclo-[4,3,0]-non-5-en. Besonders bevorzugt verwendet man ein Gemisch aus Triethylamin und dem hochnucleophilen 4-Dimethylaminopyridin.

Die Reaktionstemperaturen können bei der Durchführung der 1. Stufe des erfindungsgemäßen Verfahrens in einem größeren Bereich variiert werden. Im allgemeinen arbeitet man zwischen −20°C und +120°C, vorzugsweise zwischen 0°C und 60°C.

Bei der Durchführung der 1. Stufe des erfindungsgemäßen Verfahrens setzt man auf 1 Mol der racemischen Ausgangsverbindungen der Formel (Ia) vorzugsweise 1 bis 1,5 Mol optisch aktives Säurehalogenid der Formel (II) und 2 bis 3 Mol Base ein. Die Isolierung des Diastereomeren-Gemisches erfolgt nach üblichen Methoden. Im allgemeinen geht man so vor, daß man nach beendeter Umsetzung mit Wasser versetzt, das entstehende Gemisch mehrfach mit einem mit Wasser wenig. mischbaren organischen Lösungsmittel extrahiert, die vereinigten organischen Phasen trocknet und unter vermindertem Druck einengt.

Die Trennung der diastereomeren Ester der Formel (III) kann nach den für derartige Zwecke geeigneten Methoden vorgenommen werden, also zum Beispiel durch fraktionierte Kristallisation oder auch mit Hilfe von chromatographischen Verfahren.

Besonders bevorzugt wendet man säulenchromatographische Trennverfahren an, wie z.B. die Hochdruckfiltration über eine Kieselgelsäule mit einem Elutionsgemisch aus Hexan, Tetrachlorkohlenstoff und Propionitril.

Als Verdünnungsmittel für die 2. Stufe (Esterverseifung) des erfindungsgemäßen Verfahrens kommen ebenfalls inerte organische Lösungsmittel in Frage. Besonders bevorzugt verwendet man Alkohole, wie beispielsweise Methanol, Ethanol oder Propanol.

Die Freisetzung des erfindungsgemäßen Wirkstoffes erfolgt in der zweiten Stufe des erfindungsgemäßen Verfahrens mit Hilfe von Basen. Bevorzugt verwendent man dabei starke wäßrige anorganische Basen, wie Natriumhydroxid oder Kaliumhydroxid in Wasser.

Die Reaktionstemperaturen können bei der Durchführung der 2. Stufe des erfindungsgemäßen Verfahrens ebenfalls in einem größeren Bereich variiert werden. Im allgemeinen arbeitet man zwischen 0°C und 80°C, vorzugsweise zwischen 10°C und 60°C.

Bei der Durchführung der 2. Stufe des erfindungsgemäßen Verfahrens setzt man auf 1 Mol des jeweiligen diastereomeren Esters der Formel (III) im allgemeinen 1 bis 3 Mol, vorzugsweise 1 bis 2 Mol Base ein.

Die Isolierung des erfindungsgemäßen Wirkstoffes erfolgt nach üblichen Methoden. Im allgemeinen verfährt man in der Weise, daß man das Reaktionsgemisch mit Wasser versetzt, dann mehrfach mit einem mit Wasser wenig mischbaren organischen Lösungsmittel extrahiert, die vereinigten organischen Phasen trocknet und durch Abziehen des Lösungsmittels unter vermindertem Druck einengt und den verbleibenden Rückstand gegebenenfalls durch Umkristallisation oder durch Waschen mit einem organischen Lösungsmittel von eventuell vorhandenen Verunreinigungen befreit.

Bei der Durchführung der zweiten Stufe des erfindungsgemäßen Verfahrens wird jeweils derjenige diastereomere Ester der Formel (III) eingesetzt, aus dem durch Behandlung mit Base der erfindungsgemäße (−)-Antipode des (E)-1-Cyclohexyl-4,4-dimethyl-3-hydroxy-2-(1,2,4-triazol-1-yl)-pent-1-ens in Freiheit gesetzt wird.

Nach dem erfindungsgemäßen Verfahren läßt sich auch der (+)-Antipode des (E)-1-Cyclohexyl-3-hydroxy-2-(1,2,4-triazol-1-yl)-pent-1-ens darstellen. Man geht dabei so vor, daß man nach der Trennung der diastereomeren Ester jeweils diejenige Verbindung der Formel (III), die den (+)-Antipoden enthält, mit Base in Gegenwart eines Verdünnungsmittels behandelt. Die Reaktionsbedingungen entsprechen denjenigen, die im Falle des erfindungsgemäßen Verfahrens bei der Durchführung der zweiten Stufe in Frage kommen.

Der erfindungsgemäße Wirkstoff greift in den Metabolismus der Pflanzen ein und kann deshalb als Wachstumsregulator eingesetzt werden.

Für die Wirkungsweise von Pflanzenwachstumsregulatoren gilt nach der bisherigen Erfahrung, daß ein Wirkstoff such mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetz werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Langerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qaulitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sie die Stimulierung des Latex-flusses bei Gummibäumen genannt.

Unter dem Einfluß von Wachstumsregulatoren kann es zur Ausbildung parthenokarper Früche kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die

zum Ablösen der Früchte erforderlichen Kräfte zu reduzieren, um eine mechanische Beerntung zu ermögliche oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentriertung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Boden Induziert werden. Hierdurch wird die Kultivierung von Pflanzen in Gebieten möglich, die hierzu normalerweise ungeeignet sind.

Der erfindungsgemäße Wirkstoff kann in die üblichen Formulierungen übergeführt werden, wie Lösungen, Emulsionen, Suspensionen, Pulver, Schäume, Pasten, Granulate, Aerosole, Feinstverkapselungen un polymeren Stoffen und in Hüllmassen für Saatgut, sowie ULV-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen oder organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten in allgemeinen zwischen 0,1 und 95 Gewichtsprozent Wirkstoff, vorzugsweise zwischen 0,5 und 90%.

Der erfindungsgemäße Wirkstoff kann in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren, wobei der (+)-Antipode des (E)-1-Cyclohexyl-4,4-dimethyl-3-hydroxy-2-(1,2,4-triazol-1-yl)-pent-1-ens ausgenommen ist.

Der Wirkstoff kann als solcher, in Form seiner Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daβ die Anwendung des Wachstumsregulators in einem bevorzugten Zeitraum vorgenommen wird, dessen genaue Abgrenzung sich nach den klimatischen und vegetativen Gegebenheiten richtet.

Die Herstellung und die Verwendung des erfindungsgemäβen Wirkstoffes werden durch die nachfolgenden Beispiele veranschaulicht.

*Herstellungsbeispiele:*

Beispiel 1

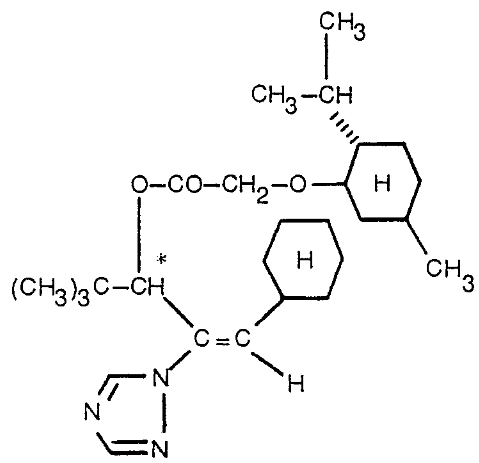

(III–1)

1. Stufe

12,0 g (0,0456 Mol) racemisches (E)-1-Cyclohexyl-4,4-dimethyl-3-hydroxy-2-(1,2,4-triazol-1-yl)-pent-1-en, 6,0 g (0.06 Mol) Triethylamin und 7,32 g (0,06 Mol) 4-Dimethylaminopyridin in 250 ml absolutem Tetrahydrofuran werden unter Rühren und Eiskühlung tropfenweise mit 11,7 g (0,0503 Mol) (−)-Menth-3-yloxyacetylchlorid versetzt und nach beendeter Zugabe weitere 20 Stunden bei 20°C bis 25°C gerührt. Zur Aufarbeitung grieβt man das Reaktionsgemisch in 500 ml Wasser, extrahiert mehrfach mit Dichlormethan, trocknet die vereinigten organischen Extrakte über Natriumsulfat und entfernt das Lösungsmittel im Vakuum.

Man erhält 18,3 g (87,2% der Theorie) an (E)-1-Cyclohexyl-4,4-dimethyl-3-(−)-(menth-3-yloxy-acetyloxy)-2-(1,2,4-triazol-1-yl)-pent-1-en Diastereomeren-Gemisch als hockviskoses Öl.

Die Trannung in die diastereomeren Einzelkomponenten erfolgt mittels HPLC* an einer Kieselgelsäule (Merck 5—20 µ Korngröβe) mit dem Elutionsmittelgemisch Hexan, Tetrachlorkohlenstoff und Propionitril (7:2:1).

Als 1. Fraktion eluiert man 4,5 g (42,9% der Theorie) an (−)-Menth-3-yloxyacetylester des (+)-Enantiomeren des (E)-1-Cyclohexyl-4,4-dimethyl-3-hydroxy-2-(1,2,4-triazol-1-yl)-pent-1-ens vom Brechungsindex $n_D^{20}$: 1,4922. Als 2. Fraktion eluiert man 4,7 g (44,9% der Theorie) an (−)-Menth-3-yloxyacetylester des (−)-Enantiomeren des (E)-1-Cyclohexyl-4,4-dimethyl-3-hydroxy-2-(1,2,4-triazol-1-yl)-pent-1-ens vom Brechungsindex $n_D^{20}$: 1,4925.

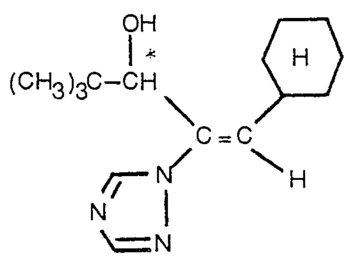

(−)–Antipode

2. Stufe

4,65 g (0,0102 Mol) der 2. Esterfraktion werden in 40 ml Methanol mit einer Lösung von 0,6 g (0,015 Mol) Natriumhydroxid in 5 ml Wasser versetzt und 3 Stunden bei 20°C bis 25°C gerührt. Zur Aufarbeitung verdünnt man mit 150 ml Wasser, extrahiert dreimal mit jeweils 120 ml Dichlormethan, wäscht die vereinigten organischen Extrakte mit Wasser, trocknet über Magnesiumsulfat und entfernt das Lösungsmittel im Vakuum. Der feste Rückstand wird mit Cyclohexan gewaschen und abgesaugt.

Man erhält 1,9 g (70,9% der Theorie) des (−)-Antipoden des (E)-1-Cyclohexyl-4,4-dimethyl-3-hydroxy-2-(1,2,4-triazol-1-yl)-pent-1-ens vom Schmelzpunkt 145° bis 148°C.

---

*=High performance liquid chormatography (Hochauflösende Flüssigkeits-Chromatographie).

Das Produkt besitzt eine optische Reinheit von 61%.
$[\alpha]_D^{20} = -48,6°$ (c=87,5 mg/10 ml $CHCl_3$)

*Vergleichsbeispiel I*

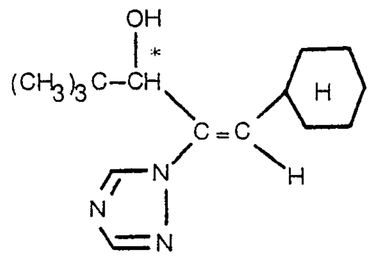

(*)—Antipode

4,49 g (0,00978 Mol) desjenigen Diastereomeren, das aus der ersten Fraktion der im Beispiel 1, erste Stufe, beschriebenen Eluierung gewonnen wurde, werden in 40 ml Methanol mit einer Lösung von 0,6 g (0,015 Mol) Natriumhydroxid in 5 ml Wasser versetzt und 3 Stunden bei 20°C bis 25°C gerührt. Zur Aufarbeitung verdünnt man mit 150 ml Wasser, extrahiert dreimal mit jeweils 120 ml Dichlormethan, wäscht die vereinigten organischen Extrakte mit Wasser, trocknet über Magnesiumsulfat und entfernt das Lösungsmittel im Vakuum. Der feste Rückstand wird mit Cyclohexan gewaschen und abgesaugt.

Man erhält 1,62 g (62,8% der Theorie) des (+)-Antipoden des (E)-1-Cyclohexyl-4,4-dimethyl-3-hydroxy-2-(1,2,4-triazol-1-yl)-pent-1-ens vom Schmelzpunkt 159—161°C.

Das Produkt besitzt eine optische Reinheit von 98%.
$[\alpha]_D^{20} = +77,3°$ (c=81,5 mg/10 ml $CHCl_3$)

*Vergleichsbeispiel II*

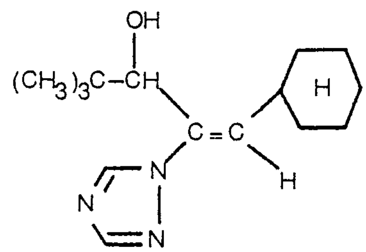

(Racemat)

26 g (0,1 Mol) (E)-1-Cyclohexyl-4,4-dimethyl-3-hydroxy-2-(1,2,4-triazol-1-yl)-pent-1-en-3-on werden in 200 ml Methanol aufgenommen und unter Rühren und Kühlen portionsweise mit 4,5 g Natriumborhydrid versetzt. Nach beendeter Reaktion wird das Reaktionsgemisch auf pH—6 eingestellt und eingeengt. Der Rückstand wird in 200 ml Methylenchlorid aufgenommen, mit gesättigter Natriumhydrogencarbonat-Lösung gewaschen, über Natriumsulfat getrocknet, filtriert und eingeengt. Der Rückstand wird aus Petrolether umkristallisiert. Man erhält 14,5 g (55% der Theorie) racemisches (E)-1-Cyclohexyl-4,4-dimethyl-3-hydroxy-2-(1,2,4-triazol-1-yl)-pent-1-en vom Schmelzpunkt 131°C.

Herstellung des Ausgangsproduktes:

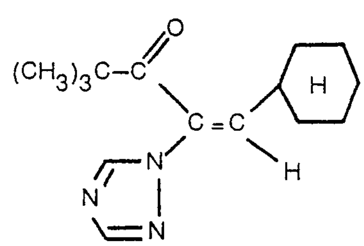

83,5 g (0,5 Mol) Pinakolyl-1,2,4-triazol, 60 g (0,54 Mol) Cyclohexanaldehyd, 4,2 g (0,05 Mol) Piperidin und 6 g (0,1 Mol) Eisessig in 300 ml Toluol werden am Wasserabscheider unter Rückfluß erhitzt, bis kein Wasser mehr übergeht. Nach dem Abkühlen der Reaktionslösung wird mit gesättigter Natriumchlorid-Lösung gewaschen, die organische Phase über Natriumsulfat getrocknet, filtriert und eingeengt. Der Rückstand wird in 500 ml Aceton aufgenommen und unter Rühren mit einer filtrierten Lösung von 90 g (0,25 Mol) Naphthalin-1,5-disulfonsäure in 500 ml Aceton versetzt.

Der zunächst ausfallende Niederschlag wird abgesaugt, das Filtrat weiter eingeengt und der erhaltene farblose kristalline Rückstand in 500 ml Methylenchlorid aufgenommen. Danach wird halbkonzentrierte wäßrige Natriumcarbonat-Lösung bis zur alkalischen Reaktion zugegeben. Die organische Phase wird abgetrennt, getrocknet, filtriert und eingeengt. Der ölige Rückstand wird in Petrolether aufgenommen, und

der Kristallisation überlassen. Man erhält 64 g (49% der Theorie) (E)-1-Cyclohexyl-4,4-dimethyl-2-(1,2,4-triazol-1-yl)-pent-1-en-3-on vom Schmelzpunkt 98°C.

*Beispiel A*

Wuchshemmung bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Wirkstoffe, Wirkstoffkonzentrationen und Versuchsergebnisse gehen aus der folgenden Tabelle hervor.

TABELLE A

Wuchshemmung Bei Gerste

| Wirkstoff | Konzentration in % | Wuchshemmung in % |
|---|---|---|
| 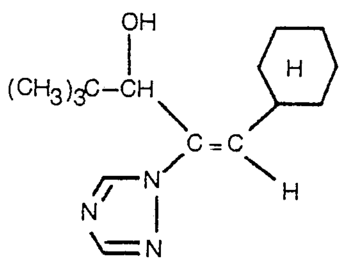 racemisch (bekannt) | 0,05 | 28 |
| 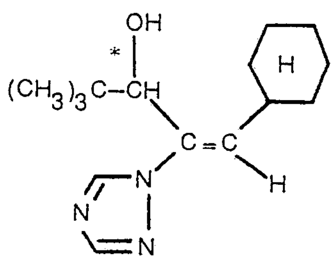 (−)-Antipode (erfindungsgemäß) | 0,05 | 38 |
| 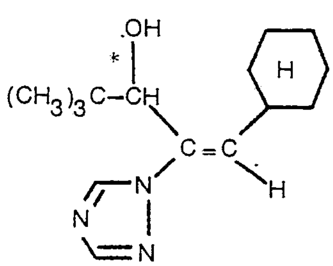 (+)-Antipode | 0,05 | 5 |

*Beispiel B*

Wuchshemmung bei Sojabohnen

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflazen werden im Gewächshaus bis zur vollen Entfaltung der ersten Folgeblattes angezoegen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Wirkstoffe, Wirkstoffkonzentrationen und Versuchsergebnisse gehen aus der folgenden Tabelle hervor.

TABELLE B

Wuchshemmung Bei Sojabohnen

| Wirkstoff | Konzentration in % | Wuchshemmung in % |
|---|---|---|
| 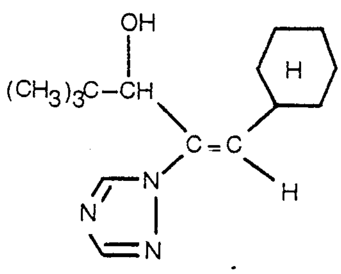 racemisch (bekannt) | 0,025 | 64 |
| 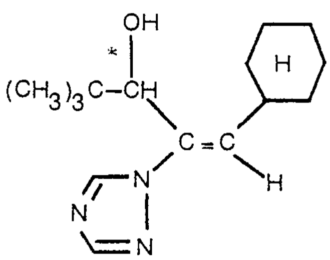 (−)-Antipode (erfindungsgemäß) | 0,025 | 77 |
| 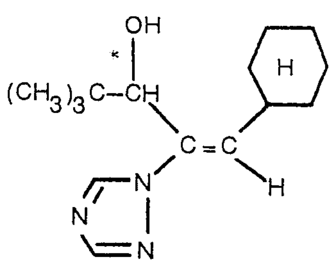 (+)-Antipode | 0,025 | 0 |

**Patentansprüche**

1. (−)-Antipode des (E)-1-Cyclohexyl-4,4-dimethyl-3-hydroxy-2-(1,2,4-triazol-1-yl)-pent-1-ens der Formel

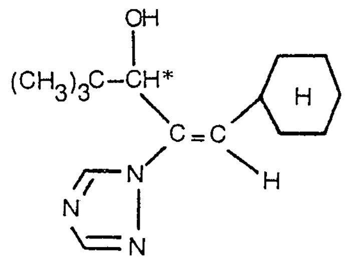

( I )

2. Verfahren zur Herstellung des (−)-Antipoden des (E)-1-Cyclohexyl-4,4-dimethyl-3-hydroxy-2-(1,2,4-triazol-1-yl)-pent-1-ens der Formel

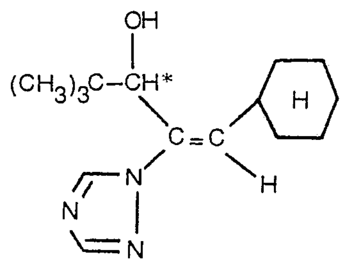

( I )

dadurch gekennzeichnet, daß man in einer ersten Stufe racemisches (E)-1-Cyclohexyl-4,4-dimethyl-3-hydroxy-2-(1,2,4-triazol-1-yl)-pent-1-en der Formel

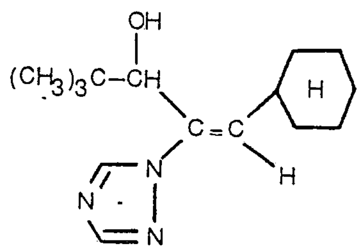

( Ia )

mit einem optisch aktiven Säurehalogenid der Formel

R—X—Hal (II)

in welcher

R für (+)-3-Brom-campher-8-yl, (+)-Campher-10-yl oder (−)-Menth-3-yl steht,

X für —CO—, $—SO_2—$ oder $—O—CH_2—CO—$ steht und

Hal für Chlor oder Brom steht,

in Gegenwart eines Verdünnungsmittels und gegebenenfalls in Gegenwart von Basen umsetzt, dann die so erhaltenen diastereomeren Ester der Formel

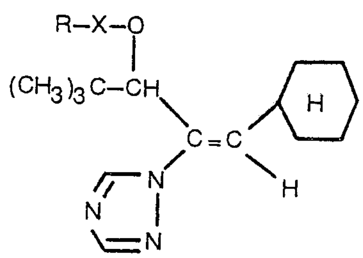

(III) (Diastereomeren-Gemisch)

in welcher

R und X die oben angegebene Bedeutung haben, aufgrund ihrer unterschiedlichen physikalischen Eigenschaften trennt,

und danach in einer zweiten Stufe den (−)-Antipoden des (E)-1-Cyclohexyl-4,4-dimethyl-3-hydroxy-2-(1,2,4-triazol-1-yl)-pent-1-ens aus dem entsprechenden Ester mit Hilfe von Basen in Gegenwart eines Verdünnungsmittels in Freiheit setzt.

3. Pflanzenwuchsregulierende Mittel, gekennzeichnet durch einen Gehalt an dem (−)-Antipoden des (E)-1-Cyclohexyl-4,4-dimethyl-3-hydroxy-2-(1,2,4-triazol-1-yl)-pent-1-ens der Formel (I), wobei ein Gehalt an dem (+)-Antipoden des (E)-1-Cyclohexyl-4,4-dimethyl-3-hydroxy-2-(1,2,4-triazol-1-yl)-pent-1-ens ausgenommen ist.

4. Verfahren zur Regulierung des Pflanzenwachstums, dadurch gekennzeichnet, daß man den (−)-Antipoden des (E)-1-Cyclohexyl-4,4-dimethyl-3-hydroxy-2-(1,2,4-triazol-1-yl)-pent-1-ens der Formel (I) auf die Pflanzen und/oder deren Lebensraum ausbringt.

5. Verwendung des (−)-Antipoden des (E)-1-Cyclohexyl-4,4-dimethyl-3-hydroxy-2-(1,2,4-triazol-1-yl)-pent-1-ens der Formel (I) zur Regulierung des Pflanzenwachstums.

6. Verfahren zur Herstellung von pflanzenwuchsregulierenden Mitteln, dadurch gekennzeichnet, daß man den (−)-Antipoden des (E)-1-Cyclohexyl-4,4-dimethyl-3-hydroxy-2-(1,2,4-triazol-1-yl)-pent-1-ens der Formel (I) mit Streckmitteln und/oder oberflächenaktiven Stoffen vermischt, wobei der (+)-Antipode des (E)-1-Cyclohexyl-4,4-dimethyl-3-hydroxy-2-(1,2,4-triazol-1-yl)-pent-1-ens als Bestandteil der Mittel ausgenommen ist.

**Revendications**

1. L'antipode lévogyre du (E)-1-cyclohexyl-4,4-diméthyl-3-hydroxy-2-(1,2,4-triazole-1-yl)-pent-1-ène de formule

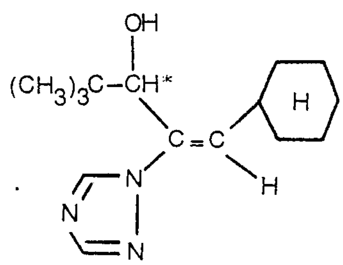

(I)

2. Procédé de préparation de l'antipode lévogyre du (E)-1-cyclohexyl-4,4-diméthyl-3-hydroxy-2-(1,2,4-triazole-1-yl)-pent-1-ène de formule

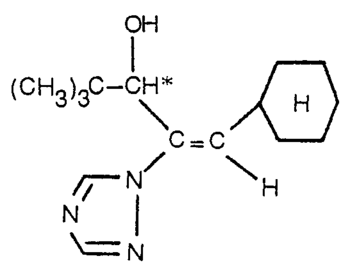

(I)

caractérisé en ce qu'on fait réagir dans une première étape le (E)-1-cyclohexyl-4,4-diméthyl-3-hydroxy-2-(1,2,4-triazole-1-yl)-pent-1-ène racémique de formule

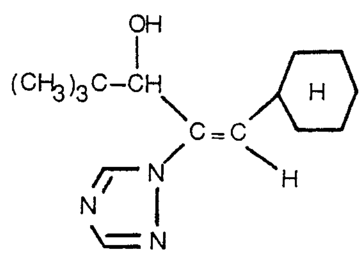

(Ia)

avec un halogénure d'acide optiquement actif de formule

R—X—Hal (II)

dans laquelle

R est le groupe (+)-3-bromo-camphre-8-yle, (+)-camphre-10-yle ou (−)-menth-3-yle,
X représente —CO—, —$SO_2$— ou —O—$CH_2$—CO—, et
Hal représente le chlore ou le brome,
en présence d'un diluant et, le cas échéant, en présence de bases, puis on sépare les esters diastéréo-isomériques ainsi obtenus de formule

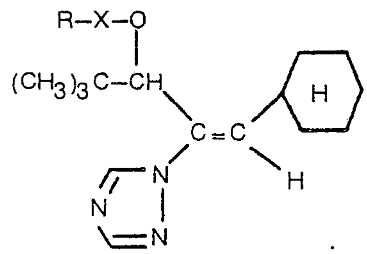

(III)
mélange de
diastéréisomères

dans laquelle
R et X ont la définition indiquée ci-dessus, sur la base de la différence de leurs propriétés physiques, après quoi, dans une seconde étape, on libère l'antipode lévogyre du (E)-1-cyclohexyl-4,4-diméthyl-3-hydroxy-2-(1,2,4-triazole-1-yl)-pent-1-ène de l'ester correspondant à l'aide de bases en présence d'un diluant.

3. Compositions influençant la croissance des plantes, caractérisées par une teneur en l'antipode lévogyre du (E)-1-cyclohexyl-4,4-diméthyl-3-hydroxy-2-(1,2,4-triazole-1-yl)-pent-1-ène de formule (I), à l'exclusion d'une teneur en l'antipode dextrogyre du (E)-1-cyclohexyl-4,4-diméthyl-3-hydroxy-2-(1,2,4-triazole-1-yl)-pent-1-ène.

4. Procédé pour influencer la croissance des plantes, caractérisé en ce qu'on applique l'antipode lévogyre du (E)-1-cyclohexyl-4,4-diméthyl-3-hydroxy-2-(1,2,4-triazole-1-yl)-pent-1-ène de formule (I) sur les plantes et/ou leur milieu.

5. Utilisation de l'antipode lévogyre du (E)-1-cyclohexyl-4,4-diméthyl-3-hydroxy-2-(1,2,4-triazole-1-yl)-pent-1-ène de formule (I) pour influencer la croissance les plantes.

6. Procédé de préparation de compositions influençant la croissance des plantes, caractérisé en ce qu'on mélange l'antipode lévogyre du (E)-1-cyclohexyl-4,4-diméthyl-3-hydroxy-2-(1,2,4-triazole-1-yl)-pent-1-ène de formule (I) avec des diluants et/ou des agents tensioactifs, en excluant l'antipode dextrogyre du (E)-1-cyclohexyl-4,4-diméthyl-3-hydroxy-2-(1,2,4-triazole-1-yl)-pent-1-ène comme composant des compositions.

**Claims**

1. (−)-antipode of (E)-1-cyclohexyl-4,4-dimethyl-3-hydroxy-2-(1,2,4-triazol-1-yl)-pent-1-ene of the formula

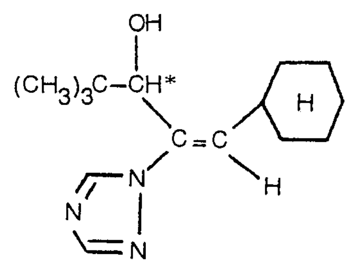

(I)

2. Process for the preparation of the (−)-antipode of (E)-1-cyclohexyl-4,4-dimethyl-3-hydroxy-2-(1,2,4-triazol-1-yl)-pent-1-ene of the formula

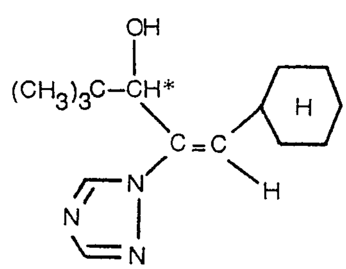

(I)

characterized in that, in a first stage, racemic (E)-1-cyclohexyl-4,4-dimethyl-3-hydroxy-2-(1,2,4-triazol-1-yl)-pent-1-ene of the formula

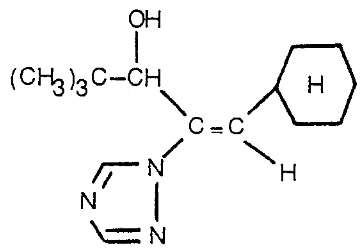

(Ia)

is reacted with an optically active acid halide of the formula

R—X—Hal (II)

in which

R represents (+)-3-bromo-campher-8-yl, (+)-campher-10-yl or (−)-menth-3-yl,

X represents —CO—, $—SO_2—$ or $—O—CH_2—CO$ and

Hal represents chlorine or bromine,

in the presence of a diluent and, if appropriate, in the presence of bases, and the resulting diastereomeric esters of the formula

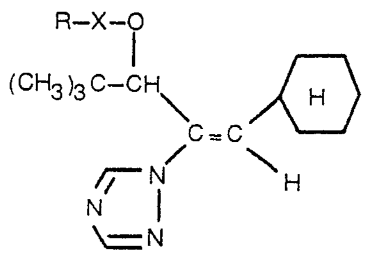

(III) (diastereomer mixture)

in which

R and X have the abovementioned meaning, are then separated on the basis of their different physical properties, and, thereafter, in a second stage, the (−)-antipode of (E)-1-cyclohexyl-4,4-dimethyl-3-hydroxy-2-(1,2,4-triazol-1-yl)-pent-1-ene is liberated from the corresponding ester with the aid of bases in the presence of a diluant.

3. Plant growth-regulating agents, characterized in that they contain the (−)-antipode of (E)-1-cyclohexyl-4,4-dimethyl-3-hydroxy-2-(1,2,4-triazol-1-yl)-pent-1-ene of the formula (I) wherein a content of (+)-antipode of (E)-1-cyclohexyl-4,4-dimethyl-3-hydroxy-2-(1,2,4-triazol-1-yl)-pent-1-ene is excluded.

4. Method of regulating plant growth, characterized in that the (−)-antipode of (E)-1-cyclohexyl-4,4-dimethyl-3-hydroxy-2-(1,2,4-triazol-1-yl)-pent-1-ene of the formula (I) is applied to the plants and/or their environment.

5. Use of the (−)-antipode of (E)-1-cyclohexyl-4,4-dimethyl-3-hydroxy-2-(1,2,4-triazol-1-yl)-pent-1-ene of the formula (I) for regulating plant growth.

6. Process for the preparation of plant growth-regulating agents, characterized in that the (−)-antipode of (E)-1-cyclohexyl-4,4-dimethyl-3-hydroxy-2-(1,2,4-triazol-1-yl)-pent-1-ene of the formula (I) is mixed with extenders and/or surface-active substances, wherein the (+)-antipode of (E)-1-cyclohexyl-4,4-dimethyl-3-hydroxy-2-(1,2,4-triazol-1-yl)-pent-1-ene is excluded as a constituent of the agents.